# EUROPÄISCHE PATENTANMELDUNG

(11) **EP 4 775 309 A1**
(43) Veröffentlichungstag der Anmeldung: **15.07.2026**
(21) Anmeldenummer: 26150550.7
(22) Anmeldetag: 07.01.2026
(51) Int. Cl.: B01L 3/00

(54) **RECYCELBARE PROBENAUFNAHMEVORRICHTUNG**

(30) Priorität: 07.01.2025 AT 500012025
(71) Anmelder: Greiner Bio-One GmbH, 4550 Kremsmünster (AT)
(72) Erfinder: Bobek, Michael, 4893 Zell am Moos (AT); Rathner, Christian, 4642 Sattledt (AT); Mizelli, Maximilian, 4551 Ried im Traunkreis (AT); Holzinger, Peter, 4600 Thalheim bei Wels (AT); Petersen, Vibeke, 4053 Haid bei Ansfelden (AT)
(74) Vertreter: Burger, Hannes Alfred

(57) **Zusammenfassung**

Die Erfindung betrifft eine Probenaufnahmevorrichtung (1) umfassend eine Kappe (2) mit einem darin angeordneten Dichtstopfen (3) und ein, mit der Kappe (2) verschließbares, insbesondere flüssigkeitsdicht und/oder luftdicht verschließbares, Probentransportröhrchen (4), wobei die Kappe (2) aus einem ersten Kunststoffmaterial und das Probentransportröhrchen (4) aus einem zweiten Kunststoffmaterial ausgebildet sind, wobei Kunststoffmaterialien für die Kappe (2) und das Probentransportröhrchen (4) Polyesterpolymere und/oder aromatische Polyesterpolymere und/oder Polyestercopolymere und/oder copolymerisiertes Polyethylenterephthalat (PET), insbesondere PETG oder PET-C, und/oder Blends dieser Materialien umfassen, wobei das erste Kunststoffmaterial und das zweite Kunststoffmaterial als Polyesterpolymer, insbesondere zu einem überwiegenden Anteil, Polyethylenterephthalat (PET) umfassen, und wobei die Kappe (2) mit einer ersten Schrumpffolie (5), insbesondere aus LDPE, im Speziellen einer, im Ausgangzustand vor dem Aufschrumpfen hohlzylindrischen ersten Schrumpffolie (5), ummantelt sein kann, wobei die erste Schrumpffolie (5) vorzugsweise farblich codiert bzw. gekennzeichnet oder eingefärbt ist.

## Beschreibung

Die Erfindung betrifft eine Probenaufnahmevorrichtung und ein Verfahren zur Herstellung einer Probenaufnahmevorrichtung.

Gattungsgemäße Probenaufnahmevorrichtungen, insbesondere Vorrichtungen zur sicheren und luftdichten Lagerung sowie zum Transport von Probenmaterialien wie Flüssigkeiten, sind aus dem Stand der Technik bekannt. Solche Vorrichtungen finden breite Anwendung in der medizinischen Diagnostik, analytischen Chemie und biologischen Forschung, wo die sichere Handhabung von Proben eine zentrale Rolle spielt. Aus dem Stand der Technik, beispielsweise RU203230U1, JPH02245660A, JP2002159475A, JP4593025B2, JP2014073871A, WO2024081240A1 und US11961422B2, sind verschiedene Probenaufnahmevorrichtungen bekannt, die jedoch die Materialkombination und Eigenschaften der Kappe und des Röhrchens nicht auf die spezifischen Anforderungen hinsichtlich dessen Recycling und gleichzeitiger chemischer Beständigkeit und Dichtigkeit optimieren.

Aufgabe der vorliegenden Erfindung war es, die Nachteile des Standes der Technik zu überwinden und eine Vorrichtung und ein Herstellungsverfahren dafür zur Verfügung zu stellen, wobei die Vorrichtung auf einfache Weise in einer Kreislaufwirtschaft integrierbar ist und weiterhin notwendige medizintechnische Anforderungen erfüllt.

Diese Aufgabe wird durch eine Vorrichtung und ein Verfahren gemäß den Ansprüchen gelöst.

Die erfindungsgemäße Probenaufnahmevorrichtung zur Probenahme von organischen Proben, insbesondere von Blut, umfasst eine Kappe mit einem darin angeordneten Dichtstopfen und ein, mit der Kappe verschließbares, insbesondere flüssigkeitsdicht und/oder luftdicht verschließbares, Probentransportröhrchen, dadurch gekennzeichnet, dass die Kappe aus einem ersten Kunststoffmaterial und das Probentransportröhrchen aus einem zweiten Kunststoffmaterial ausgebildet sind, wobei Kunststoffmaterialien für die Kappe und das Probentransportröhrchen Polyesterpolymere und/oder aromatische Polyesterpolymere und/oder Polyestercopolymere und/oder copolymerisiertes Polyethylenterephthalat (PET), insbesondere PETG oder PET-C, und/oder Blends dieser Materialien umfassen, wobei das erste Kunststoffmaterial und das zweite Kunststoffmaterial als Polyesterpolymer, insbesondere zu einem überwiegenden Anteil, Polyethylenterephthalat (PET) umfassen.

Insbesondere durch die Verwendung von copolymerisiertem PET, insbesondere PETG oder PET-C, weist die jeweilige Komponente der Probenaufnahmevorrichtung eine verbesserte Beständigkeit gegen Peracetic Acid (PAA; Handelsname Proxitane 15:23) auf. Diese Verbindung kann insbesondere zum Reinigen der Komponenten eingesetzt werden.

Die Probenaufnahmevorrichtung ist damit in einem mehrstufigen Recyclingprozess recycelbar sein. In einer ersten Stufe wird die Probenaufnahmevorrichtung geschreddert bzw. zerkleinert, gewaschen und dekontaminiert, damit organische Reststoffe entfernt werden können. Zum Entfernen dieser organischen Reststoffe kann beispielsweise Peracetic Acid PAA (Handelsname Proxitane 14:23) eingesetzt. In einer zweiten Stufe wird das nunmehr zerkleinerte und gewaschene Kunststoffmaterial der Probenaufnahmevorrichtung mit einem Float-Sink-Verfahren getrennt, um die einzelnen Materialkomponenten wiederverwenden zu können bzw. einer sortenreinen Wiederaufbereitung zuführen zu können. Das Float-Sink-Verfahren ist eine Trennmethode, die auf der Dichteunterschiede von Materialien basiert.

Des Weiteren kann es zweckmäßig sein, wenn die Kappe mit einer ersten Schrumpffolie, insbesondere aus Polyethylen mit niedriger Dichte (LDPE), im Speziellen einer, im Ausgangzustand vor dem Aufschrumpfen hohlzylindrischen ersten Schrumpffolie, ummantelt ist, wobei die erste Schrumpffolie vorzugsweise farblich codiert bzw. gekennzeichnet oder eingefärbt ist.

Ferner kann vorgesehen sein, dass das Probentransportröhrchen mit einer zweiten Schrumpffolie, insbesondere aus LDPE, im Speziellen einer, im Ausgangszustand vor dem Aufschrumpfen hohlzylindrischen zweiten Schrumpffolie, ummantelt ist, wobei die zweite Schrumpffolie vorzugsweise transparent ist.

Die Probenaufnahmevorrichtung ist damit in einem mehrstufigen Recyclingprozess recycelbar sein. In einer ersten Stufe wird die Probenaufnahmevorrichtung geschreddert bzw. zerkleinert, gewaschen und dekontaminiert, damit organische Reststoffe entfernt werden können. Zum Entfernen dieser organischen Reststoffe kann beispielsweise Peracetic Acid PAA (Handelsname Proxitane 14:23) eingesetzt. In einer zweiten Stufe wird das nunmehr zerkleinerte und gewaschene Kunststoffmaterial der Probenaufnahmevorrichtung mit einem Float-Sink-Verfahren getrennt, um die einzelnen Materialkomponenten wiederverwenden zu können bzw. einer sortenreinen Wiederaufbereitung zuführen zu können. Das Float-Sink-Verfahren ist eine Trennmethode, die auf der Dichteunterschiede von Materialien basiert. Im Recyclingprozess von PET, das mit einer leichteren Schrumpffolie überzogen ist, kann das Verfahren beispielhaft folgendermaßen durchgeführt werden. Nach dem Zerkleinern der Probenaufnahmevorrichtung und Entfernen der organischen Reste bzw. Stoffe können die zerkleinerten Partikel in ein Wasserbad oder ein anderes Flüssigkeitsmedium mit einer definierten Dichte eingebracht werden. Für PET wird typischerweise ein Medium mit einer Dichte zwischen der des PET (ca. 1,2-1,58 g/cm³) und der leichteren Schrumpffolie (z. B. LDPE mit ca. 0,91-0,93 g/cm³) bzw. des Dichtstopfens verwendet. Im Wasserbad sinken die PET-Partikel aufgrund ihrer höheren Dichte nach unten, während die Schrumpffolie sowie andere leichtere Kunststoffe oder Reststoffe, wie beispielsweise eines, auf die Schrumpffolie aufgebrachten Etiketts, an der Oberfläche schwimmen. Ein derartiges Etikett ist im klinischen Alltag bei Verwendung des Probenaufnahmevorrichtung zur Kennzeichnung derselben notwendig und wird im Allgemeinen mittels einer Klebeschicht an dem Probentransportröhrchen festgelegt. Wäre ein derartiges Etikett direkt am PET des Probentransportröhrchens angebracht, so wäre eine aufwendige Trennung des Etiketts und des Klebstoffes der Klebeschicht vom PET notwendig, um das so recycelte PET in einer Kreislaufwirtschaft zu verwenden. Durch Zwischenschalten der Schrumpffolie kann somit eben diese Schrumpffolie gemeinsam mit dem Etikett und dessen Klebeschicht einfach vom PET des Probentransportröhrchens entfernt werden, was den Recycling-Prozess des PET verbessert. Auf diese Weise lassen sich PET und Schrumpffolie oder weitere Störstoffe mechanisch voneinander trennen. Die schwimmenden Folien oder Störstoffe werden von der Oberfläche abgeschöpft, während das sinkende PET gesammelt wird, um im nächsten Schritt weiterverarbeitet zu werden. Dieses Verfahren ermöglicht eine effiziente Trennung von PET und leichteren Kunststofffolien, was die Reinheit des PET-Recyclats erhöht und den Recyclingprozess insgesamt vereinfacht.

Darüber hinaus kann vorgesehen sein, dass das Probentransportröhrchen eine hohlzylinderförmige Behälterwand und einen daran anschließenden kugelkalottenförmigen Boden aufweist, wobei die Behälterwand wenigstens in einem Beschichtungsbereich außerhalb eines Überdeckungsbereiches der Kappe mit dem Probentransportröhrchen vollständig mit der zweiten Schrumpffolie ummantelt ist, wobei insbesondere der Boden außerhalb des Beschichtungsbereiches definiert ist.

Vorteilhaft ist auch eine Ausprägung, gemäß welcher vorgesehen sein kann, dass die erste Schrumpffolie und die zweite Schrumpffolie im Ausgangszustand als einstückige gemeinsame Schrumpffolie verbunden ausgebildet sind, wobei die gemeinsame Schrumpffolie einen Kappenbereich und einen Röhrchenbereich umfasst, wobei der Röhrchenbereich zum Beschichtungsbereich aufschrumpfbar korrespondierend ausgebildet ist, und wobei der Kappenbereich entlang einer Trennlinie mittels Perforationen vom Röhrchenbereich beim Aufschrumpfen trennbar ist. Die Trennlinie mit Perforationen erlaubt eine präzise und effiziente Trennung von Kappen- und Röhrchenbereich beim Aufschrumpfen, was die Produktionszeit und die Kosten senkt.

Gemäß einer Weiterbildung ist es möglich, dass die Schrumpffolie Materialkomponenten umfasst, die durch Energieeintrag mittels Laserstrahl farbumschlagend sind. Die farbumschlagenden Materialkomponenten der Schrumpffolie ermöglichen eine einfache und schnelle optische Kennzeichnung der Probenbehälter durch Lasergravur. Dies erleichtert die Identifikation und Rückverfolgbarkeit im Laborbetrieb.

Ferner kann es zweckmäßig sein, wenn das erste Kunststoffmaterial, insbesondere bei und/oder vor Herstellung des Probentransportröhrchens durch ein Spritzgussverfahren, eine Intrinsische Viskosität mit einem Wert von weniger als 0,6 dl/g aufweist. Die geringe intrinsische Viskosität des Kunststoffmaterials für die Kappe verbessert die Formbarkeit und ermöglicht eine präzisere Fertigung. Dies trägt zur Dichtigkeit und zur optimalen Passform der Kappe bei.

Darüber hinaus kann vorgesehen sein, dass das zweite Kunststoffmaterial, insbesondere bei und/oder vor Herstellung der Kappe durch ein Spritzgussverfahren, eine intrinsische Viskosität mit einem Wert von weniger als 0,55 dl/g aufweist. Ein Material mit niedriger intrinsischer Viskosität erleichtert die Verarbeitbarkeit beim Spritzgussverfahren, was die Herstellung des Probentransportröhrchens effizienter und kostengünstiger macht.

Des Weiteren kann vorgesehen sein, dass das erste Kunststoffmaterial und das zweite Kunststoffmaterial gleich sind. Die Verwendung des gleichen Materials reduziert die Komplexität im Recyclingprozess, da keine zusätzliche materialbedingte Sortierung erforderlich ist. Dies erleichtert die Wiederverwertung und erhöht die Effizienz im Kreislaufwirtschaftssystem.

Gemäß einer besonderen Ausprägung ist es möglich, dass der Dichtstopfen eine Dichte mit einem geringeren Wert, im Spezielle einen um 0,1 g/ cm³ geringeren Wert, als die Dichte der Kappe und/oder des Probentransportröhrchens, insbesondere mit einem um wenigstens 0,1 g/ cm³ geringeren Wert als die Dichte von PET, im Speziellen kleiner als 1,25 g/cm³, insbesondere aus einem Bereich umfassend 0,91 g/cm³ bis 1,15 g/cm³ aufweist, wobei der Dichtstopfen insbesondere aus Polyisopropen mit einer Dichte von etwa 1,1 g/cm³ ausgebildet ist. Die geringere Dichte des Dichtstopfens erleichtert die Trennung im Recyclingprozess, da der Stopfen beim Float-Sink-Verfahren an der Oberfläche bleibt, während Kappe und Röhrchen sinken, wobei dafür jedenfalls eine Dichte-Differenz von 0,1 g/ cm³ vorteilhaft ist. Dies verbessert die Reinheit des PET-Recyclats. Insbesondere ist es vorteilhaft, wenn der Dichtstopfen eine Dichte aus einem Bereich umfassend 0,91 g/cm³ bis 1,15 g/cm³ oder wenigstens aus einem Bereich umfassend 0,91 g/cm³ bis 1,1 g/cm³ aufweist, sodass im Float-Sink-Verfahren vorteilhafterweise Wasser oder eine Kochsalzlösung, insbesondere eine 15%-ige Kochsalzlösung, oder eine Lösung mit anderen Salzen als Medium bzw. Trennmedium verwendbar ist. In diesem Zusammenhang wäre es jedoch auch denkbar, dass der Dichtstopfen eine höhere Dichte aufweist, die aber jedenfalls kleiner als die Dichte von PET (ca. 1,2-1,58 g/cm³) ist. In diesem Fall kann eine Kochsalzlösung als Trenn-Medium vorgesehen werden, dessen Dichte zwischen der des PET und der des Dichtstopfens liegt, sodass weiterhin ein Float-Sink-Verfahren anwendbar ist.

Entsprechend einer vorteilhaften Weiterbildung kann vorgesehen sein, dass das erste Kunststoffmaterial und/oder das zweite Kunststoffmaterial einen Zusatzstoff umfasst, sodass die Säurebeständigkeit erhöht ist.

Um die Säurebeständigkeit von PET bzw. des ersten und des zweiten Kunststoffmaterials zu verbessern und gleichzeitig die Transparenz zu erhalten, könnten die nachfolgend beschriebenen Stabilisatoren und Zusatzstoffe eingesetzt werden. Antioxidantien können die Abbaurate von PET durch säureinduzierte Oxidation verringern. Phosphit-basierte Antioxidantien (z. B. Tris(nonylphenyl)phosphit oder TNPP) sowie hinderlich substituierte Phenole wie Irganox 1010 sind dafür bekannt, oxidative Schäden zu reduzieren, ohne die Transparenz des Materials wesentlich zu beeinflussen. UV-Stabilisatoren wie Benzotriazole und Hindered Amine Light Stabilizers (HALS) können die Zersetzung von PET durch Licht und Säureeinwirkung verlangsamen. Sie sind besonders hilfreich, wenn das PET im Außenbereich oder unter Lichteinwirkung verwendet wird, da sie die chemische Degradation minimieren und transparent bleiben. Beispiele sind Tinuvin 328 oder Chimassorb 944. Organosilane bzw. ein Silan-basiertes Additiv, wie z. B. Alkoxysilane, können durch Vernetzung auf der Oberfläche eine Schutzschicht bilden, die gegen chemische Einwirkungen resistent ist und die Transparenz bewahrt. Diese Silan-basierten Additive können die Oberfläche von PET hydrophober machen und so die Anfälligkeit gegenüber Säuren reduzieren. Transparente Nanopartikel, wie Silica (SiO₂)- oder Aluminiumoxid (Al₂O₃)-Nanopartikel, können in geringen Konzentrationen in die PET-Matrix eingebracht werden, um eine physische Barriere zu schaffen. Diese feinen Partikel erhöhen die chemische Stabilität des PET und beeinträchtigen bei niedrigen Konzentrationen kaum die Lichtdurchlässigkeit. Solche Nanokomposite werden oft zur Verstärkung von PET verwendet, da sie zudem die mechanische Festigkeit erhöhen. Additive wie Fluorpolymere (z. B. Teflon-Partikel in nanoskaliger Form) oder spezielle Polysiloxane (hydrophobe Additive) können auf der PET-Oberfläche eine hydrophobe Schicht bilden, die die Benetzung durch Säuren und die Anfälligkeit für chemische Angriffe verringert. Fluorpolymere sind besonders effektiv, können jedoch leicht die Kosten erhöhen und sollten transparent genug formuliert sein. Die Zugabe von Ionomeren, wie Ethylen-Methacrylsäure-Copolymeren, die in eine klare PET-Matrix integriert sind, kann die chemische Beständigkeit verbessern, indem sie die Wechselwirkungen des Polymers mit Säuren reduzieren. Diese Ionomere können als Barriere wirken, ohne die Transparenz des PET wesentlich zu beeinträchtigen.

Diese möglichen Zusatzstoffe können in kleinen Konzentrationen eingesetzt werden, um die optischen Eigenschaften des PET nicht zu beeinträchtigen und dennoch weitere vorteilhafte Eigenschaften des PET bzw. des ersten und zweiten Kunststoffmaterial zu bedingen. Der Zusatzstoff erhöht die chemische Beständigkeit der Materialien gegen aggressive Reinigungsmittel wie Peracetic Acid (PAA). Dies sorgt für eine sichere Wiederverwendbarkeit.

Die Erfindung betrifft weiter noch ein Verfahren zur Herstellung einer Probenaufnahmevorrichtung, umfassend die folgenden Verfahrensschritte:
- Bereitstellen der Probenaufnahmevorrichtung in zusammengebautem bzw. zusammengesetztem zustand, wobei die Probenaufnahmevorrichtung eine Kappe mit einem darin angeordneten Dichtstopfen und ein, mit der Kappe verschlossenes, insbesondere flüssigkeitsdicht und/oder luftdicht verschlossenes, Probentransportröhrchen umfasst,
   -- wobei die Kappe insbesondere aus einem ersten Kunststoffmaterial und das Probentransportröhrchen insbesondere aus einem zweiten Kunststoffmaterial ausgebildet sind, wobei Kunststoffmaterialien für die Kappe und das Probentransportröhrchen Polyesterpolymere und/oder aromatische Polyesterpolymere und/oder Polyestercopolymere und/oder copolymerisiertes Polyethylenterephthalat (PET), insbesondere PETG oder PET-C, und/oder Blends dieser Materialien umfassen, wobei das erste Kunststoffmaterial und das zweite Kunststoffmaterial als Polyesterpolymer, insbesondere zu einem überwiegenden Anteil,

Polyethylenterephthalat (PET) umfasst;
- Bereitstellen einer ersten Schrumpffolie, insbesondere aus LDPE, im Speziellen einer, im Ausgangzustand vor dem Aufschrumpfen hohlzylindrischen ersten Schrumpffolie;
- Ummanteln der Kappe mit der ersten Schrumpffolie, durch Aufschrumpfen der ersten Schrumpffolie, wobei die Kappe mit der ersten Schrumpffolie insbesondere farblich codiert bzw. gekennzeichnet oder eingefärbt wird.

Das Verfahren ermöglicht eine individuelle farbliche Codierung der Kappe, was die Differenzierung und Zuordnung von Proben erleichtert. Dies verbessert die Arbeitsabläufe in Laboren und Krankenhäusern.

Insbesondere kann es vorteilhaft sein, wenn weiters das Bereitstellen einer zweiten Schrumpffolie, insbesondere aus LDPE, im Speziellen einer, im Ausgangszustand vor dem Aufschrumpfen hohlzylindrischen zweiten Schrumpffolie, und das Ummanteln eines Beschichtungsbereiches des Probentransportröhrchens mit der zweiten Schrumpffolie vorgesehen sind, wobei insbesondere ein, der Kappe gegenüberliegender Boden des Probentransportröhrchens beschichtungsfrei ist. Somit kann ein Etikett auf das Probentransportröhrchen, also insbesondere auf die Schrumpffolie des Probentransportröhrchens, aufgebracht werden, dass in einem Recyclingprozess einfach wieder entfernbar ist. Da Etiketten vorwiegend mit Klebstoff bzw. einer Klebeschicht an einem Probentransportröhrchen angebracht werden, würde dieser Klebstoff bei direkter Verbindung mit dem PET des Probentransportröhrchens eine aufwendige Trennung bedingen. Durch Zwischenschalten der Schrumpffolie kann somit eben diese Schrumpffolie gemeinsam mit dem Etikett und dessen Klebeschicht einfach vom PET des Probentransportröhrchens entfernt werden, was den Recycling-Prozess des PET verbessert.

Ferner kann vorgesehen sein, dass die erste Schrumpffolie und die zweite Schrumpffolie im Ausgangszustand, also im nicht aufgeschrumpften Zustand, als einstückige gemeinsame Schrumpffolie verbunden ausgebildet bereitgestellt werden, wobei die gemeinsame Schrumpffolie einen Kappenbereich und einen Röhrchenbereich umfasst, wobei der Röhrchenbereich zum Beschichtungsbereich aufschrumpfbar korrespondierend ausgebildet ist und im Beschichtungsbereich aufgeschrumpft wird, und wobei der Kappenbereich entlang einer Trennlinie mittels Perforationen vom Röhrchenbereich beim Aufschrumpfen, insbesondere durch Aufschrumpfen mit Wärmeeinwirkung auf die gemeinsame Schrumpffolie, getrennt wird.

Das Trennen der Schrumpffolie durch Wärmeeinwirkung während des Aufschrumpfens ermöglicht eine präzise Anpassung an die Kappe und an das Röhrchen. Dies sorgt für eine optimale Passform und reduziert die Wahrscheinlichkeit von Materialdefekten.

Zum besseren Verständnis der Erfindung wird diese anhand der nachfolgenden Figur näher erläutert.

Es zeigt in stark vereinfachter, schematischer Darstellung:
- Fig. 1: eine Probenaufnahmevorrichtung mit Kappe und Dichtstopfen;
- Fig. 2: die Progenaufnahmevorrichtung vor dem Ummanteln mit einer gemeinsamen Schrumpffolie.

Einführend sei festgehalten, dass in den unterschiedlich beschriebenen Ausfüh-rungsformen gleiche Teile mit gleichen Bezugszeichen bzw. gleichen Bauteilbe-zeichnungen versehen werden, wobei die in der gesamten Beschreibung enthal-tenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind die-se Lageangaben bei einer Lageänderung sinngemäß auf die neue Lage zu über-tragen.

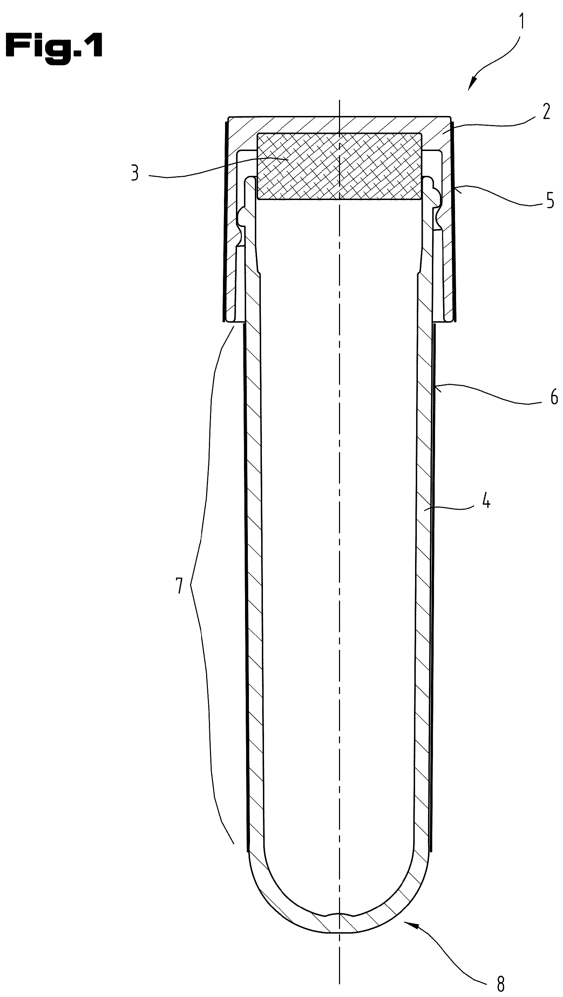

In Fig. 1 ist eine mögliche Ausgestaltungsform der Probenaufnahmevorrichtung 1 mit einer Kappe 2, einem Dichtstopfen 3 und ein, mit der Kappe 2 verschließbares, insbesondere flüssigkeitsdicht und/oder luftdicht verschließbares, Probentransportröhrchen 4 in stark vereinfachter, schematischer Darstellung. Dabei ist die Kappe 2 aus einem ersten Kunststoffmaterial und das Probentransportröhrchen 4 aus einem zweiten Kunststoffmaterial ausgebildet, wobei Kunststoffmaterialien für die Kappe 2 und das Probentransportröhrchen 4 Polyesterpolymere und/oder aromatische Polyesterpolymere und/oder Polyestercopolymere und/oder copolymerisiertes Polyethylenterephthalat (PET), insbesondere PETG oder PET-C, und/oder Blends dieser Materialien umfassen, wobei das erste Kunststoffmaterial und das zweite Kunststoffmaterial als Polyesterpolymer, insbesondere zu einem überwiegenden Anteil, Polyethylenterephthalat (PET) umfassten.

Es kann vorgesehen sein, dass die Kappe 2 mit einer ersten Schrumpffolie 5, insbesondere aus LDPE, im Speziellen einer, im Ausgangzustand vor dem Aufschrumpfen hohlzylindrischen ersten Schrumpffolie 5, ummantelt ist, wobei die erste Schrumpffolie 5 vorzugsweise farblich codiert bzw. gekennzeichnet oder eingefärbt ist. Weiters kann vorgesehen sein, dass das Probentransportröhrchen 4 mit einer zweiten Schrumpffolie 6, insbesondere aus LDPE, im Speziellen einer, im Ausgangszustand vor dem Aufschrumpfen hohlzylindrischen zweiten Schrumpffolie 6, ummantelt ist, wobei die zweite Schrumpffolie 6 vorzugsweise transparent ist.

In der Fig. ist eine weitere Darstellung der Ausführungsform der Probenaufnahmevorrichtung 1 gezeigt, wobei wiederum für gleiche Teile gleiche Bezugszeichen bzw. Bauteilbezeichnungen wie in der vorangegangenen Fig. 1 verwendet werden. Um unnötige Wiederholungen zu vermeiden, wird auf die detaillierte Beschreibung in der vorangegangenen Fig. 1 hingewiesen bzw. Bezug genommen.

Hinsichtlich der Ummantelung mit der ersten Schrumpffolie 5 und der zweiten Schrumpffolie 6 kann vorgesehen sein, dass das diesbezügliche Verfahren zu Herstellung der Probenaufnahmevorrichtung 1 die folgenden Verfahrensschritte umfasst:
- Bereitstellen der Probenaufnahmevorrichtung 1 in zusammengebautem bzw. zusammengesetztem zustand, wobei die Probenaufnahmevorrichtung 1 eine Kappe 2 mit einem darin angeordneten Dichtstopfen 3 und ein, mit der Kappe 2 verschlossenes, insbesondere flüssigkeitsdicht und/oder luftdicht verschlossenes, Probentransportröhrchen 4 umfasst,
   -- wobei die Kappe 2 insbesondere aus einem ersten Kunststoffmaterial und das Probentransportröhrchen 4 insbesondere aus einem zweiten Kunststoffmaterial ausgebildet sind, wobei Kunststoffmaterialien für die Kappe 2 und das Probentransportröhrchen 4 Polyesterpolymere und/oder aromatische Polyesterpolymere und/oder Polyestercopolymere und/oder copolymerisiertes PET, insbesondere PETG oder PET-C, und/oder Blends dieser Materialien umfassen, wobei das erste Kunststoffmaterial und das zweite Kunststoffmaterial als Polyesterpolymer, insbesondere zu einem überwiegenden Anteil, Polyethylenterephthalat (PET) umfasst;
- Bereitstellen der ersten Schrumpffolie 5, insbesondere aus LDPE, im Speziellen einer, im Ausgangzustand vor dem Aufschrumpfen hohlzylindrischen ersten Schrumpffolie 5;
- Ummanteln der Kappe 2 mit der ersten Schrumpffolie 5, durch Aufschrumpfen der ersten

Schrumpffolie 5, wobei die Kappe 2 mit der ersten Schrumpffolie 5 insbesondere farblich codiert bzw. gekennzeichnet oder eingefärbt wird.

Diesbezüglich kann es zweckmäßig sein, wenn weiters das Bereitstellen der zweiten Schrumpffolie 6, insbesondere aus LDPE, im Speziellen einer, im Ausgangszustand vor dem Aufschrumpfen hohlzylindrischen zweiten Schrumpffolie 6, und das Ummanteln eines Beschichtungsbereich 7 des Probentransportröhrchens 4 mit der zweiten Schrumpffolie 6 vorgesehen sind, wobei insbesondere ein, der Kappe 2 gegenüberliegender Boden 8 des Probentransportröhrchens 4 beschichtungsfrei ist.

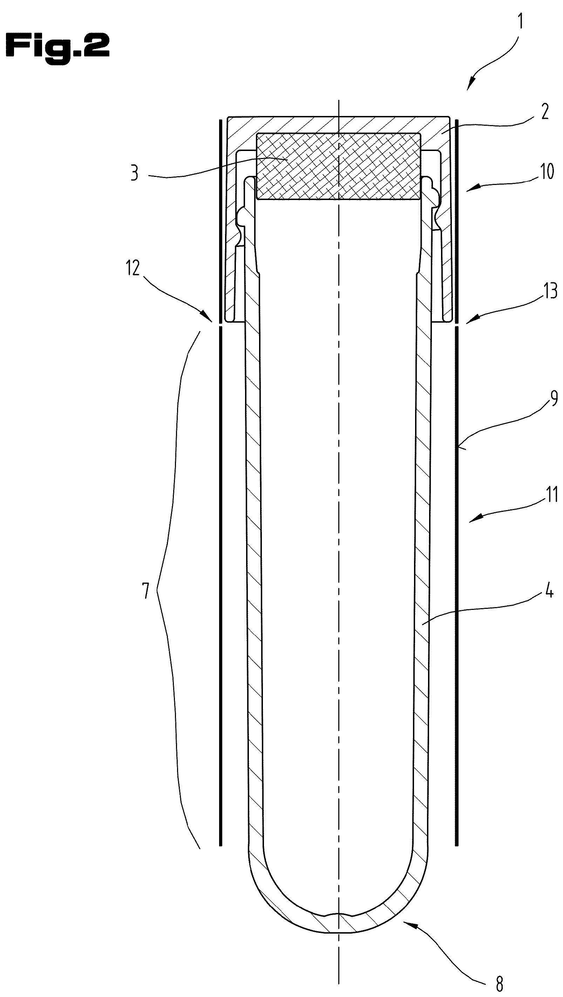

Wie aus Fig. 2 ersichtlich, kann es zweckmäßig sein, wenn die erste Schrumpffolie 5 und die zweite Schrumpffolie 6 im Ausgangszustand, also im nicht aufgeschrumpften Zustand, als einstückige gemeinsame Schrumpffolie 9 verbunden ausgebildet bereitgestellt werden, wobei die gemeinsame Schrumpffolie 9 einen Kappenbereich 10 und einen Röhrchenbereich 11 umfasst, wobei der Röhrchenbereich 11 zum Beschichtungsbereich 7 aufschrumpfbar korrespondierend ausgebildet ist und im Beschichtungsbereich 7 aufgeschrumpft wird, und wobei der Kappenbereich 10 entlang einer Trennlinie 12 mittels Perforationen 13 vom Röhrchenbereich 11 beim Aufschrumpfen, insbesondere durch Aufschrumpfen mit Wärmeeinwirkung auf die gemeinsame Schrumpffolie 9, getrennt wird.

Als sinnvolle Weiterbildung der Schrumpffolie 5, 6 bzw. 9 kann vorgesehen sein, dass die Schrumpffolie 5, 6 bzw. 9 Materialkomponenten umfasst, die durch Energieeintrag mittels Laserstrahl farbumschlagend sind, sodass die Schrumpffolie 5, 6 bzw. 9 mittels Laserstrahl beschriftbar oder kennzeichenbar ist.

Das Probentransportröhrchens 4 und die Kappe 2 werden vorzugsweise durch Spritzgussverfahren hergestellt, wobei insbesondere für die Kappe 2 vorgesehen sein kann, das zweite Kunststoffmaterial eine intrinsische Viskosität mit einem Wert von weniger als 0,55 dl/g aufweist, sodass die Kappe vollständig ausformbar ist und eine geringe Schrumpfung aufweist. Hinsichtlich Produktions- und Recyclingkosten wäre es denkbar vorteilhaft, wenn das erste Kunststoffmaterial und das zweite Kunststoffmaterial gleich sind.

Hinsichtlich des Recyclingprozesses bzw. dem Eingliedern der Probenaufnahmevorrichtung 1 in eine Material-Wiederaufbereitung kann insbesondere vorgesehen sein, dass der Dichtstopfen 3 eine Dichte mit einem geringeren Wert, im Spezielle einen um 0,1 g/ cm³ geringeren Wert, als die Dichte der Kappe 2 und/oder des Probentransportröhrchens 4, insbesondere mit einem geringeren Wert als die Dichte von PET, im Speziellen kleiner als 1 g/cm³, insbesondere aus einem Bereich umfassend 0,91 g/cm³ bis 1,15 g/cm³ aufweist. Dies kann insbesondere bei Verwendung eines Float-Sink-Verfahren als Teilverfahren einer Material-Wiederaufbereitung vorteilhaft sein.

Die Ausführungsbeispiele zeigen mögliche Ausführungsvarianten, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Schutzbereich ist durch die Ansprüche bestimmt. Die Beschreibung und die Zeichnungen sind jedoch zur Auslegung der Ansprüche heranzuziehen. Einzelmerkmale oder Merkmalskombinationen aus den gezeigten und beschriebenen unterschiedlichen Ausführungsbeispielen können für sich eigenständige erfinderische Lösungen darstellen. Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Der Ordnung halber sei abschließend darauf hingewiesen, dass zum besseren Verständnis des Aufbaus Elemente teilweise unmaßstäblich und/oder vergrößert und/oder verkleinert dargestellt wurden.

### Bezugszeichenaufstellung

- 1: Probenaufnahmevorrichtung
- 2: Kappe
- 3: Dichtstopfen
- 4: Probentransportröhrchen
- 5: Erste Schrumpffolie
- 6: Zweite Schrumpffolie
- 7: Beschichtungsbereich
- 8: Boden
- 9: Gemeinsame Schrumpffolie
- 10: Kappenbereich
- 11: Röhrchenbereich
- 12: Trennlinie
- 13: Perforationen

## Patentansprüche

1. Probenaufnahmevorrichtung (1) umfassend eine Kappe (2) mit einem darin angeordneten Dichtstopfen (3) und ein, mit der Kappe (2) verschließbares, insbesondere flüssigkeitsdicht und/oder luftdicht verschließbares, Probentransportröhrchen (4), **dadurch gekennzeichnet, dass** die Kappe (2) aus einem ersten Kunststoffmaterial und das Probentransportröhrchen (4) aus einem zweiten Kunststoffmaterial ausgebildet sind, wobei Kunststoffmaterialien für die Kappe (2) und das Probentransportröhrchen (4) Polyesterpolymere und/oder aromatische Polyesterpolymere und/oder Polyestercopolymere und/oder copolymerisiertes Polyethylenterephthalat (PET), insbesondere PETG oder PET-C, und/oder Blends dieser Materialien umfassen, wobei das erste Kunststoffmaterial und das zweite Kunststoffmaterial als Polyesterpolymer, insbesondere zu einem überwiegenden Anteil, Polyethylenterephthalat (PET) umfassen.

2. Probenaufnahmevorrichtung (1) nach einem der vorhergehenden Ansprüche, **dadurch gekennzeichnet, dass** die Kappe (2) mit einer ersten Schrumpffolie (5), insbesondere aus LDPE, im Speziellen einer, im Ausgangzustand vor dem Aufschrumpfen hohlzylindrischen ersten Schrumpffolie (5), ummantelt ist, wobei die erste Schrumpffolie (5) vorzugsweise farblich codiert bzw. gekennzeichnet oder eingefärbt ist.

3. Probenaufnahmevorrichtung (1) nach einem der vorhergehenden Ansprüche, **dadurch gekennzeichnet, dass** das Probentransportröhrchen (4) mit einer zweiten Schrumpffolie (6), insbesondere aus LDPE, im Speziellen einer, im Ausgangszustand vor dem Aufschrumpfen hohlzylindrischen zweiten Schrumpffolie (6), ummantelt ist, wobei die zweite Schrumpffolie (6) vorzugsweise transparent ist.

4. Probenaufnahmevorrichtung (1) nach Anspruch 3, **dadurch gekennzeichnet, dass** das Probentransportröhrchen (4) eine hohlzylinderförmige Behälterwand und einen daran anschließenden kugelkalottenförmigen Boden (8) aufweist, wobei die Behälterwand wenigstens in einem Beschichtungsbereich (7) außerhalb eines Überdeckungsbereiches der Kappe (2) mit dem Probentransportröhrchen (4) vollständig mit der zweiten Schrumpffolie (6) ummantelt ist, wobei insbesondere der Boden (8) außerhalb des Beschichtungsbereiches (7) definiert ist.

5. Probenaufnahmevorrichtung (1) nach Anspruch 2 und 4, **dadurch gekennzeichnet, dass** die erste Schrumpffolie (5) und die zweite Schrumpffolie (6) im Ausgangszustand als einstückige gemeinsame Schrumpffolie (9) verbunden ausgebildet sind, wobei die gemeinsame Schrumpffolie (9) einen Kappenbereich (10) und einen Röhrchenbereich (11) umfasst, wobei der Röhrchenbereich (11) zum Beschichtungsbereich (7) aufschrumpfbar korrespondierend ausgebildet ist, und wobei der Kappenbereich (10) entlang einer Trennlinie (12) mittels Perforationen (13) vom Röhrchenbereich (11) beim Aufschrumpfen trennbar ist.

6. Probenaufnahmevorrichtung (1) nach einem der Ansprüche 2 bis 5, **dadurch gekennzeichnet, dass** die Schrumpffolie (5, 6 bzw. 9) Materialkomponenten umfasst, die durch Energieeintrag mittels Laserstrahl farbumschlagend sind.

7. Probenaufnahmevorrichtung (1) nach einem der vorhergehenden Ansprüche, **dadurch gekennzeichnet, dass** das erste Kunststoffmaterial, insbesondere bei und/oder vor Herstellung des Probentransportröhrchens (4) durch ein Spritzgussverfahren, eine Intrinsische Viskosität mit einem Wert von weniger als 0,6 dl/g aufweist.

8. Probenaufnahmevorrichtung (1) nach einem der vorhergehenden Ansprüche, **dadurch gekennzeichnet, dass** das zweite Kunststoffmaterial, insbesondere bei und/oder vor Herstellung der Kappe (2) durch ein Spritzgussverfahren, eine intrinsische Viskosität mit einem Wert von weniger als 0,55 dl/g aufweist.

9. Probenaufnahmevorrichtung (1) nach einem der vorhergehenden Ansprüche, **dadurch gekennzeichnet, dass** das erste Kunststoffmaterial und das zweite Kunststoffmaterial gleich sind.

10. Probenaufnahmevorrichtung (1) nach einem der vorhergehenden Ansprüche, **dadurch gekennzeichnet, dass** der Dichtstopfen (3) eine Dichte mit einem geringeren Wert, im Spezielle einen um 0,1 g/ cm³ geringeren Wert, als die Dichte der Kappe (2) und/oder des Probentransportröhrchens (4), insbesondere mit einem um wenigstens 0,1 g/ cm³ geringeren Wert als die Dichte von PET, im Speziellen kleiner als 1,25 g/cm³, insbesondere aus einem Bereich umfassend 0,91 g/cm³ bis 1,15 g/cm³ aufweist, wobei der Dichtstopfen (3) insbesondere aus Polyisopropen mit einer Dichte von etwa 1,1 g/cm³ ausgebildet ist.

11. Probenaufnahmevorrichtung (1) nach einem der vorhergehenden Ansprüche, **dadurch gekennzeichnet, dass** das erste Kunststoffmaterial und/oder das zweite Kunststoffmaterial einen Zusatzstoff umfasst, sodass die Säurebeständigkeit erhöht ist.

12. Verfahren zur Herstellung einer Probenaufnahmevorrichtung (1), umfassend die folgenden Verfahrensschritte:
- Bereitstellen der Probenaufnahmevorrichtung (1) in zusammengebautem bzw. zusammengesetztem zustand, wobei die Probenaufnahmevorrichtung (1) eine Kappe (2) mit einem darin angeordneten Dichtstopfen (3) und ein, mit der Kappe (2) verschlossenes, insbesondere flüssigkeitsdicht und/oder luftdicht verschlossenes, Probentransportröhrchen (4) umfasst,
-- wobei die Kappe (2) insbesondere aus einem ersten Kunststoffmaterial und das Probentransportröhrchen (4) insbesondere aus einem zweiten Kunststoffmaterial ausgebildet sind, wobei Kunststoffmaterialien für die Kappe (2) und das Probentransportröhrchen (4) Polyesterpolymere und/oder aromatische Polyesterpolymere und/oder Polyestercopolymere und/oder copolymerisiertes PET, insbesondere PETG oder PET-C, und/oder Blends dieser Materialien umfassen, wobei das erste Kunststoffmaterial und das zweite Kunststoffmaterial als Polyesterpolymer, insbesondere zu einem überwiegenden Anteil, Polyethylenterephthalat (PET) umfasst;
- Bereitstellen einer ersten Schrumpffolie (5), insbesondere aus LDPE, im Speziellen einer, im Ausgangzustand vor dem Aufschrumpfen hohlzylindrischen ersten Schrumpffolie (5);
- Ummanteln der Kappe (2) mit der ersten Schrumpffolie (5), durch Aufschrumpfen der ersten Schrumpffolie (5), wobei die Kappe (2) mit der ersten Schrumpffolie (5) insbesondere farblich codiert bzw. gekennzeichnet oder eingefärbt wird.

13. Verfahren nach Anspruch 12, **dadurch gekennzeichnet, dass** weiters das Bereitstellen einer zweiten Schrumpffolie (6), insbesondere aus LDPE, im Speziellen einer, im Ausgangszustand vor dem Aufschrumpfen hohlzylindrischen zweiten Schrumpffolie (6), und das Ummanteln eines Beschichtungsbereiches (7) des Probentransportröhrchens (4) mit der zweiten Schrumpffolie (6) vorgesehen sind, wobei insbesondere ein, der Kappe (2) gegenüberliegender Boden (8) des Probentransportröhrchens (4) beschichtungsfrei ist.

14. Verfahren nach Anspruch 13, **dadurch gekennzeichnet, dass** die erste Schrumpffolie (5) und die zweite Schrumpffolie (6) im Ausgangszustand, also im nicht aufgeschrumpften Zustand, als einstückige gemeinsame Schrumpffolie (9) verbunden ausgebildet bereitgestellt werden, wobei die gemeinsame Schrumpffolie (9) einen Kappenbereich (10) und einen Röhrchenbereich (11) umfasst, wobei der Röhrchenbereich (11) zum Beschichtungsbereich (7) aufschrumpfbar korrespondierend ausgebildet ist und im Beschichtungsbereich (7) aufgeschrumpft wird, und wobei der Kappenbereich (10) entlang einer Trennlinie (12) mittels Perforationen (13) vom Röhrchenbereich (11) beim Aufschrumpfen, insbesondere durch Aufschrumpfen mit Wärmeeinwirkung auf die gemeinsame Schrumpffolie (9), getrennt wird.
